# FASCICULE DE BREVET EUROPEEN

(11) **EP 0 730 657 B1**
(45) Date de publication et mention de la délivrance du brevet: **30.04.2008**
(21) Numéro de dépôt: 95902161.9
(22) Date de dépôt: 22.11.1994
(51) Int. Cl.: C12N 15/86, C12N 5/10, A61K 48/00

(54) **COMPOSITION POUR LA PRODUCTION DE PRODUITS THERAPEUTIQUES IN VIVO**
ZUSAMMENSETZUNG ZUR IN VIVO ERZEUGUNG VON THERAPEUTISCHEN PRODUKTEN
COMPOSITION FOR THE IN VIVO PRODUCTION OF THERAPEUTIC PRODUCTS

(30) Priorité: 23.11.1993 FR 9313977
(43) Date de publication de la demande: 11.09.1996
(73) Titulaire: Aventis Pharma S.A., 92160 Antony (FR); INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: BEUZARD, Yves, F-75007 Paris (FR); DANOS, Olivier, F-92380 Garches (FR); DESCAMPS, Vincent, F-78160 Marly-le-Roi (FR); HEARD, Jean-Michel, F-75003 Paris (FR); MOULLIER, Philippe, F-92100 Meudon (FR); NAFFAKH, Nadia, F-92240 Malakoff (FR); PERRICAUDET, Michel, F-28320 Ecrosnes (FR); VAINCHENKER, William, F-75005 Paris (FR)
(74) Mandataire: Dernoncour, Roxane
(86) Numéro de dépôt international: PCT/FR1994/001359
(87) Numéro de publication internationale: WO 1995/014785

(56) Documents cités:
- WO-A-89/07944
- WO-A-93/06223
- M/S-M¹DICINE SCIENCES, vol.9, no.2, Février 1993, HEIDELBERG,GERMANY pages 208 - 210 DANOS ET AL 'R¹IMPLANTATION DE CELLULES G¹N¹TIQUEMENT MODIFI¹ES DANS DES N¹O-ORGANES VASCULARIS¹S'
- JOURNAL OF CELLULAR BIOCHEMISTRY, vol.0,16PT F, 1992, NEW YORK,USA page 62 MOULLIER ET AL 'MURINE SKIN FIBROBLASTS AND BONE MARROW CELLS AS TARGETS FOR IN VIVO TRANSFER AND EXPRESSION OF THE HUMAN BETA-GLUCORONIDASE CDNA'
- JOURNAL OF CELLULAR BIOCHEMISTRY, vol.S15F, 1991, NEW YORK,USA page 242 MOULLIER ET AL 'ORGANOID NEOVASCULAR STRUCTURE:EFFECTS OF VARIOUS MATRIX AND ANGIOGENIC FACTORS'
- FILE SERVER STN KARLSRUHE,FILE MEDLINE ABSTRACT NO.91284450 & CLIN EXP METASTASIS, (1991 MAY-JUN) 9(3) 231-43

## Description

La présente invention concerne le domaine de la thérapie génique et cellulaire. Plus particulièrement, elle concerne des compositions cellulaires destinées à être implantées in vivo, pour délivrer de manière prolongée et contrôlée des substances thérapeutiques.

La thérapie génique consiste à corriger une déficience ou une anomalie (mutation, expression aberrante, etc) par introduction d'une information génétique dans un organisme affecté. Cette information génétique peut être introduite soit in vitro dans une cellule extraite de l'organisme, la cellule modifiée étant alors réintroduite dans l'organisme, soit directement in vivo dans le tissu approprié. Différentes techniques ont été décrites pour l'introduction de cette information génétique, parmi lesquelles des techniques diverses de transfection impliquant des complexes d'ADN et de DEAE-dextran [Pagano et al., J.Virol. 1 (1967) 891], d'ADN et de protéines nucléaires [Kaneda et al., Science 243 (1989) 375], d'ADN et de lipides [Felgner et al., PNAS 84 (1987) 7413], l'emploi de liposomes [Fraley et al., J.Biol.Chem. 255 (1980) 10431], etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

Les techniques impliquant l'administration directe du gène in vivo présentent certains inconvénients, tels que la dispersion non sélective du gène dans tout l'organisme, sa faible demi-vie, les risques de réaction immunologique, ou encore le manque de contrôle du gène après injection. Pour cette raison, la thérapie cellulaire offre une alternative interessante, consistant à prélever les cellules, à les modifier ex vivo, puis à les réadministrer. De ce fait, seule une population de cellules identifiée est modifiée par le gène thérapeutique. Toutefois, après administration, le devenir des cellules modifiées n'est pas contrôlé. De même, il n'est plus possible d'arrêter le traitement. Enfin, cette thérapie implique que les cellules à traiter puissent être prélevées de l'organisme, manipulées ex vivo, et facilement réimplantées. De ce fait, elle est pratiquement limitée aux cellules du sang.

La présente invention apporte une solution avantageuse à ces problèmes. La présente invention concerne en effet des compositions destinées à être implantées dans un organisme, comprenant des cellules modifiées par un adénovirus recombinant défectif comprenant une séquence d'ADN hétérologue codant pour un produit thérapeutique, un gélifiant, et un support sur lequel sont ancrées lesdites cellules.

L'implantation des compositions selon l'invention offre de nombreux avantages par rapport à l'art antérieur, et en particulier le contrôle du nombre de cellules implantées, le contrôle du nombre de cellules infectées, la mesure du taux d'expression du gène thérapeutique avant implantation, l'absence de réaction immunologique liée à l'injection directe d'un virus, la possibilité de retirer l'implant à tout moment, etc.

L'implantation de cellules modifiées génétiquement a déjà été envisagée dans l'art antérieur. Ainsi, Palmer et al. [PNAS 88 (1991) 1330] et Moullier et al. [Nature genetics 4 (1993) 154] ont décrit l'implantation de fibroblastes modifiés génétiquement par des rétrovirus. Le document WO 890713 décrit un système dans lequel des hépatocytes transduits par un vecteur rétroviral comportant un gène d'intérêt thérapeutique sont ensuite implantés in vivo. Le document Médecine Sciences (1993) 9: 208-210 décrit un système dans lequel des cellules dont modifiées par un vecteur rétroviral comprenant un gène hétérologue puis incluses dans un gel de collagène contenant des fibres synthétiques en présence de facteurs de croissance. Toutefois, l'utilisation de rétrovirus pose certains problèmes limitant les applications de cette technologie. Notamment, les rétrovirus sont difficiles à produire avec des titres élevés, et, de ce fait, ne permettent pas l'utilisation à des multiplicités d'infections élevées. Les rétrovirus présentent également l'inconvénient de ne pas pouvoir incorporer de fragments d'ADN hétérologue de taille importante, limitant de ce fait les applications thérapeutiques. Enfin, les rétrovirus s'intègrent dans le génome des fibroblastes, ce qui peut contribuer à l'apparition de cellules tumorales après implantation. Par ailleurs, les fibroblastes décrits par Palmer et al. sont enrobés uniquement dans du collagène, et les implants obtenus n'ont pas une cohésion suffisante. De ce fait, ils se désagrègent progressivement in vivo, entraînant une diffusion incontrôlée des cellules hors du site d'implantation.

Les compositions selon l'invention permettent de résoudre ces inconvénients. La présente invention résulte en partie de la mise en évidence que les adénovirus sont capables in vitro d'infecter avec des pouvoirs très élevés des cellules en culture. Ainsi, il est possible d'infecter 100 % des fibroblastes en culture. Par ailleurs, il est également possible, en variant la multiplicité d'infection, d'obtenir un nombre de copies élevé d'adénovirus par cellule (jusqu'à 100 copies), ce qui permet d'augmenter l'effet thérapeutique des implants de l'invention de manière importante. De plus, les adénovirus de l'invention peuvent être produits avec des titres élevés, ce qui permet d'infecter non seulement des cultures primaires de cellules, mais également des cultures secondaires, préalablement clonées et conservées. Les adénovirus de l'invention présentent également l'avantage de ne pas s'intégrer dans le génome des cellules qu'ils infectent. De ce fait, les implants de l'invention sont moins susceptibles d'induire l'apparition de cellules tumorales. De plus, si les cellules implantées se divisent, l'adénovirus de l'invention sera dilué au cours des générations, et le caractère qu'il confère aux cellules infectées ne sera pas transmis aux cellules filles. Enfin, les vecteurs adénoviraux utilisés dans la présente invention peuvent être modifiés de façon à incorporer des fragments d'ADN hétérologue de très grande taille. Ainsi, contrairement aux autres vecteurs viraux, il est par exemple possible d'incorporer un gène hétérologue de grande taille tel celui du facteur VIII ou de la dystrophine. De plus, il est possible d'incorporer, en plus du gène thérapeutique, un gène de sécurité dont l'expression permettrait par exemple de détruire la cellule infectée.

Les compositions selon l'invention présentent donc de nombreux avantages par rapport aux systèmes décrits dans l'art antérieur, qui leur confèrent des potentialités thérapeutiques très supérieures.

Les compositions selon l'invention peuvent être réalisées à partir de différents types de cellules, et notamment à partir de fibroblastes, de cellules endothéliales, épithéliales, gliales, d'hépatocytes, de kératinocytes ou encore de myoblastes. Préférentiellement, on utilise dans le cadre de l'invention des fibroblastes.

Dans un mode préféré de réalisation de l'invention, on utilise des cellules autologues, c'est-à-dire prélevées à partir du patient chez lequel elles seront ensuite implantées. Cependant, dans certains cas, il peut être intéressant d'utiliser des cellules allogéniques ou xénogéniques, entraînant un rejet progressif de l'implant et ainsi, lui donnant un effet limité dans le temps. En particulier, des cellules d'origine murine peuvent être implantées chez l'homme sans autre effet que le rejet progressif.

Les cellules utilisées dans le cadre de l'invention peuvent être des cultures primaires. Dans ce cas, elles peuvent être prélevées par toute technique connue de l'homme du métier, puis mises en culture dans des conditions permettant leur prolifération. S'agissant plus particulièrement de fibroblastes, ceux-ci peuvent être aisément obtenus à partir de biopsies, par exemple selon la technique décrite par Ham [Methods Cell.Biol. 21a (1980) 255]. Ces cellules peuvent être utilisées directement pour la préparation des compositions de l'invention, ou conservées, par exemple par congélation, pour l'établissement de banques autologues, en vue d'une utilisation ultérieure. Préférentiellement, les compositions selon l'invention comprennent 10⁵ à 10¹⁰ cellules. Plus préférentiellement, elles en comprennent 10⁶ à 10⁸.

Les cellules en culture sont ensuite infectées par des adénovirus recombinants, pour leur conférer des propriétés thérapeutiques recherchées. L'infection est réalisée in vitro selon des techniques connues de l'homme du métier. En particulier, selon le type de cellules utilisé et le nombre de copies de virus par cellule désiré, l'homme du métier peut adapter la multiplicité d'infection et éventuellement le nombre de cycles d'infection réalisé. Il est bien entendu que ces étapes doivent être effectuées dans des conditions de stérilité appropriées pour une administration in vivo des compositions obtenues.

Les doses d'adénovirus recombinant utilisées pour l'infection des cellules peuvent être adaptées en fonction de différents paramètres, et notamment en fonction du type cellulaire, de la pathologie concernée, du gène à exprimer, ou encore de la durée du traitement recherchée. D'une manière générale, les adénovirus recombinants selon l'invention sont utilisés pour l'infection à des doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée (par exemple la lignée 293), et mesure, généralement après 2 à 4 jours, du nombre de plages de cellules infectées. Les techniques de détermination du titre pfu d'une solution virale sont bien documentées dans la littérature.

Les adénovirus recombinants utilisés dans le cadre de la présente invention sont défectifs, c'est-à-dire incapables de se répliquer de façon autonome dans la cellule infectée. Généralement, le génome des adénovirus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence d'ADN hétérologue. Préférentiellement, le virus défectif conserve néanmoins les séquences de son génome qui sont nécessaires à l'encapsidation des particules virales.

Il existe différents sérotypes d'adénovirus, dont la structure et les propriétés varient quelque peu. Néanmoins, ces virus ne sont pas pathogènes pour l'homme, et notamment les sujets non immuno-déprimés. Parmi ces sérotypes, on préfère utiliser dans le cadre de la présente invention les adénovirus humains de type 2 ou 5 (Ad 2 ou Ad 5) ou les adénovirus d'origine animale (voir demande FR 93 05954). Parmi les adénovirus d'origine animale utilisables dans le cadre de la présente invention on peut citer les adénovirus d'origine canine, bovine, murine, [exemple : Mav1, Beard et al., Virology 75 (1990) 81], ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple].

De préférence, on utilise dans le cadre de l'invention des adénovirus d'origine humaine, canine ou mixte, c'est-à-dire comprenant des régions issues d'un adénovirus humain et des régions issues d'un adénovirus canin.

Les adénovirus recombinants défectifs selon l'invention peuvent être préparés par toute technique connue de l'homme du métier [Levrero et al., Gene 101 (1991) 195, EP 185 573; Graham, EMBO J. 3 (1984) 2917]. En particulier, ils peuvent être préparés par recombinaison homologue entre un adénovirus et un plasmide portant entre autre la séquence d'ADN hétérologue. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 [Graham et al., J. Gen. Virol. 36 (1977) 59] qui contient notamment, intégrée dans son génome, la partie gauche du génome d'un adénovirus Ad5 (12 %). Des stratégies de construction de vecteurs dérivés des adénovirus ont également été décrites dans les demandes n° FR93 05954 et FR 93 08596 qui sont incorporées à la présente demande par référence.

Ensuite, les adénovirus qui se sont multipliés sont récupérés et purifiés selon les techniques classiques de biologie moléculaire, comme illustré dans les exemples.

Comme indiqué ci-dessus, les adénovirus recombinants utilisés dans le cadre de la présente invention comprennent une séquence d'ADN hétérologue codant pour un produit thérapeutique. Le produit thérapeutique peut être tout ARN, peptide, polypeptide ou protéine dont la production dans l'organisme est recherchée.

De préférence, la séquence d'ADN hétérologue comprend également des signaux d'expression permettant la production du produit thérapeutique dans les cellules infectées. Il peut s'agir des séquences qui sont naturellement responsables de l'expression de ce produit thérapeutique lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs E1A, MLP, CMV, RSV, PGK, etc. En outre, ces séquences d'expression peuvent être modifiées par addition de séquences d'activation, de régulation, etc. Par ailleurs, lorsque le gène inséré ne comporte pas de séquences d'expression, il peut être inséré dans le génome du virus défectif en aval d'une telle séquence.

Plus préférentiellement, la séquence d'ADN hétérologue comprend des signaux permettant la production et la sécrétion du produit thérapeutique par les cellules infectées implantées. En effet, la néo-vascularisation des implants selon l'invention permet une libération particulièrement efficace des produits thérapeutiques dans la circulation, libération qui est de plus prolongée et contrôlée. A cet effet, la séquence d'ADN hétérologue comporte généralement, en amont du gène thérapeutique, une séquence signal dirigeant le produit thérapeutique synthétisé dans les voies de sécrétion de la cellule infectée. Cette séquence signal peut être la séquence signal naturelle du produit thérapeutique, mais il peut également s'agir de toute autre séquence signal fonctionnelle dans la cellule infectée, ou d'une séquence signal artificielle.

Avantageusement, le produit thérapeutique est choisi parmi les enzymes (tels que notamment la superoxide dismutase, la catalase, les amylases, les lipases, les amidases, la chymosine, etc), les dérivés sanguins (tels que la sérum-albumine, l'alpha- ou la béta-globine, le facteur VII, le facteur VIII, le facteur IX, le facteur de von Willebrand, la fibronectine, l'alpha-1 antitrypsine, etc), l'insuline et ses variants, les lymphokines (telles que les interleukines, les interférons, les facteurs de stimulation des colonies [G-CSF, GM-CSF, M-CSF, SCF, ...], le TNF, le TRF, etc), les facteurs de croissance (tels que l'hormone de croissance, l'érythropoiétine, l'hormone para-thyroïdienne, le FGF, l'EGF, le PDGF, le TGF, le BDNF, le NGF, le CNTF, etc), les apolipoprotéines, ou encore des polypeptides antigéniques pour la réalisation de vaccins (hépatite, cytomégalovirus, Epstein-Barr, herpes, etc).

Comme indiqué ci-dessus, les implants selon l'invention sont particulièrement avantageux en ce sens qu'ils permettent de contrôler la libération du produit thérapeutique dans l'organisme : Celle-ci est tout d'abord déterminée par la multiplicité d'infection et par le nombre de cellules implantées. Ensuite, la libération peut être contrôlée soit par le retrait de l'implant, ce qui arrête définitivement le traitement, soit par l'utilisation de systèmes d'expression régulables, permettant d'induire ou de réprimer l'expression des gènes thérapeutiques.

Pour la préparation des compositions selon l'invention, différents types de gélifiants peuvent être employés. Les gélifiants sont utilisés pour favoriser l'ancrage des cellules sur le support par inclusion des cellules dans une matrice ayant la constitution d'un gel. Différents agents d'adhésion cellulaire peuvent donc être utilisés, tels que notamment le collagène, la gélatine, les glycosaminoglycans, la fibronectine, les lectines, etc. De préférence, on utilise dans le cadre de la présente invention du collagène. Il peut s'agir de collagène d'origine humaine, bovine ou murine. Plus préférenciellement, on utilise du collagène de type I ou III.

Comme indiqué ci-avant, les compositions selon l'invention comprennent également un support permettant l'ancrage des cellules. Le terme ancrage désigne toute forme d'interaction biologique et/ou chimique et/ou physique entraînant l'adhésion et/ou la fixation des cellules sur le support. Par ailleurs, les cellules peuvent soit recouvrir le support utilisé, soit pénétrer à l'intérieur de ce support, soit les deux. On préfère utiliser dans le cadre de l'invention un support solide, non toxique et/ou bio-compatible. En particulier, on peut utiliser des fibres de polytétrafluoroéthylène (PTFE). Dans un mode particulier de mise en oeuvre de l'invention, on utilise un support d'origine biologique, tel que notamment le collagène réticulé, la poudre d'os, les polymères à base d'hydrates de carbone et les supports à base de calcaire.

La présente invention a également pour objet un procédé de préparation d'une composition telle que définie précédemment selon lequel on effectue les étapes suivantes :
a) on isole et cultive in vitro les cellules d'un échantillon de tissu,
b) on infecte les cellules obtenues en a) avec un adénovirus recombinant comprenant un séquence d'ADN hétérologue codant pour un produit thérapeutique,
c) on incube les cellules infectées avec un milieu contenant un gélifiant,
d) on dépose le mélange obtenu en c) sur un support, éventuellement après recouvrement de celui-ci par le gélifiant,
e) on incube le mélange obtenu en d) dans des conditions permettant la gélification du gélifiant et l'ancrage des cellules sur le support, et
f) on récupère la composition obtenue.

Comme indiqué précédemment, l'étape a) du procédé peut être évitée si l'on travaille à partir de cultures secondaires de cellules ou à partir de cellules extraites de banques cellulaires. Plus préférentiellement, avant l'étape c), les cellules infectées sont lavées plusieurs fois. Ensuite, pour la préparation de l'implant, les cellules infectées sont cultivées en présence du gelifiant, puis, lorsque la matrice est formée, elle est déposée sur le support éventuellement après mise en contact de celui-ci avec le gélifiant. De plus, les cellules ou le support peuvent être mis en présence également de facteurs de croissance, tels que des facteurs angiogéniques, favorisant la formation de l'implant. Dans ce cas, des traces de facteur de croissance peuvent subsister dans l'implant. Le mélange support/gélifiant/cellules est ensuite incubé pendant une période suffisante pour permettre la gélification du gélifiant, puis la matrice ainsi obtenue est maintenue dans du milieu de culture pour permettre aux cellules de s'ancrer sur le support. L'implant ainsi préparé peut être implanté directement dans l'organisme.

Des méthodes de préparation d'implants ont également été décrites par Moullier et al [Nature Genetics 4 (1993) 154] qui peuvent être adaptées par l'homme du métier aux implants selon l'invention (voir aussi FR 93 09185 et FR 93 04700).

L'invention concerne également une méthode pour le traitement de maladies comprenant l'implantation d'une composition telle que définie ci-avant capable de produire un produit thérapeutique apte à corriger ladite maladie. Préférentiellement, cette méthode est applicable au traitement de maladies résultant d'une déficience en un produit thérapeutique et l'implant est capable de produire ledit produit. Encore plus préférentiellement, la méthode selon l'invention est utilisable pour le traitement de la thalassémie, de déficiences en érythropoiétine (insuffisance rénale, etc), en hormone de croissance, en apolipoprotéines, en hormone thyroïdienne, en facteurs de coagulation, etc. Avantageusement, la méthode selon l'invention comprend l'implantation au niveau de la cavité péritonéale, dans le tissu sous-cutané (région sus-pubienne, fosses iliaques ou inguinales, etc), dans un organe, un muscle, une tumeur, le système nerveux central, ou encore sous une muqueuse.

La présente invention sera plus complètement décrite à l'aide des exemples qui suivent, qui doivent être considérés comme illustratifs et non limitatifs.

### EXEMPLES

### 1. Techniques générales

### 1.1. Isolement et culture de fibroblastes

Les fibroblastes ont été obtenus à partir de biopsies cutanées réalisées dans des conditions stériles. Pour cela, un ou plusieurs fragments de peau sont prélevés, hachés et soumis à une digestion enzymatique. A cet effet, les lambeaux sont incubés à 37°C sous agitation douce dans un milieu DMEM supplémenté par 2 mM de L-glutamine, de la streptomycine et du sérum de veau fétal (250 µg/ml). Au bout de 2 heures, la solution est centrifugée à 1500 t/mn et le culot cellulaire est lavé 2 fois dans le même milieu dépourvu d'enzymes. Les cellules sont ensuite comptées et ensemencées, à raison de 4.10⁶ cellules vivantes/flacon, dans dés flacons contenant environ 25 ml du même milieu,

### 1.2. Infection des fibroblastes par un adénovirus recombinant

L'infection des fibroblastes avec un adénovirus recombinant est avantageusement effectuée avec une solution d'adénovirus purifiée, par exemple sur chlorure de césium (Cf exemple 2.2.). L'intérêt d'un tel protocole réside notamment dans le fait que, contrairement aux rétrovirus, un seul cycle d'infection suffit pour obtenir un pourcentage très élevé de cellules infectées (jusqu'à 100 %). De plus, l'intérêt des adénovirus réside également dans le fait que des multiplicités d'infection très grandes peuvent être employées (1000 pfu par cellule par exemple).

Généralement, les cellules à confluence sont incubées en présence d'une solution concentrée renfermant une quantité déterminée d'adénovirus recombinant. La multiplicité d'infection peut être adaptée par l'homme du métier en fonction des cas. Au bout d'une heure environ, du surnageant de culture est ajouté et les cellules sont maintenues pour la nuit. Ensuite, les cellules sont lavées dans un milieu dépourvu de virus, récoltées et de nouveau lavées. Les cellules sont ensuite incorporées dans le milieu contenant le gélifiant. Le pourcentage d'infection est alors contrôlé. Bien que cela ne soit généralement pas nécessaire, il est ensuite possible d'effectuer d'autres cycles d'infection pour augmenter le nombre de copies par cellules. Par ailleurs, ce protocole peut être appliqué à d'autres types cellulaires tels que les cellules endothéliales, épithéliales, gliales, les hépatocytes, les kératinocytes ou les myoblastes.

### 1.3. Préparation des implants

Le support utilisé (support synthétique ou biologique) est stérilisé, de préférence par autoclavage, puis recouvert de collagène de type I. Plus particulièrement, s'agissant de fibres de polytétrafluoroéthylène (Gore et Associates), l'addition de collagène est effectuée en plongeant pendant 1 heure environ les fibres dans une solution comportant du collagène [solution d'acide acétique 0,1N contenant 0,1 % de collagène de rat (sigma)]. De préférence, cette opération est réalisée sous vide afin de chasser l'air présent dans les fibres. Le support ainsi obtenu, recouvert d'un film de collagène, est transféré dans des boites de pétri et séché. Ensuite, une solution comprenant des facteurs de croissance dilués dans un tampon phosphate est ajoutée (10-20 ng de FGF basique pour 50 mg de fibres). Le support est ensuite placé dans des puits ou boites dont la taille correspond à celle des implants recherchés. Les cellules infectées par les adénovirus recombinants sont mises en suspension dans du milieu RPMI comprenant du collagène et du FGF basique. Cette suspension cellulaire est alors déposée sur le support. Après gélification du collagène (environ 30 minutes à 37°C sous 5 % CO2), les lattices de gel sont détachées des puits à l'aide d'une aiguille, puis maitenues à température ambiante 48 à 96 heures selon la vitesse de rétraction (le milieu est changé au bout de 48 heures). La composition obtenue est prête à être implantée.

### 1.4. Implantation

Les compositions décrites ci-dessus sont implantées sous anesthésie dans les zones intrapéritonéales les mieux vascularisée, par exemple au niveau du tablier épiploïque. Un ou plusieurs implants peuvent être introduits dans le même organisme au même site ou en des sites différents (jusqu'à 10 par exemple, selon le gène thérapeutique, la pathologie, la taille des implants, etc) Après implantation, il est possible de réaliser une bourse de manière à maintenir le ou les implants, et à faciliter leur excision. Généralement, des connexions vasculaires s'établissent dès les premiers jours, permettant une libération efficace du produit thérapeutique.

Il est également possible d'implanter les compositions de l'invention dans le tissu sous-cutané (région sus-pubienne, fosses iliaques ou inguinales, etc), dans un organe, un muscle ou encore sous une muqueuse.

### 2. Construction d'un adénovirus recombinant comprenant une séquence d'ADN hétérologue codant pour l'érythropoiétine.

L'érythropoiétine (Epo) est une hormone spécifique de la prolifération terminale et de la différenciation des précurseurs des globules rouges. Son gène a été cloné, séquencé, et exprimé in vitro (EP 148 605). L'Epo recombinante est largement utilisée pour remplacer le déficit de production par le rein, en cas d'insuffisance rénale chronique. A forte dose, l'Epo a également un intérêt thérapeutique potentiel pour les anémies génétiquement déterminées (B thalassémie, dépranocytose) et certains déficits de l'hématopoièse. Pour l'ensemble de ces applications, la production prolongée et contrôlée d'Epo au moyen d'un implant selon l'invention constitue un progrès important et permet de réduire les coûts prohibitifs des doses élevées d'Epo recombinante.

### 2.1. Construction du plasmide pAd.RSV.Epo

Le plasmide pAd.RSV.Epo a été construit à partir du plasmide pAd.RSV.βGal [Stratford-Perricaudet et al., J. Clin. Invest. (1992) 626] par substitution du gène B-gal par le gène de l'Epo.

Pour cela, le cDNA codant pour l'Epo de singe et contenant son propre signal de sécrétion a été isolé à partir du plasmide Bluescript sous forme d'un fragment XhoI-EcoRV. Ce fragment a ensuite été cloné aux sites SalI (situé après le LTR RSV) et EcoRV (situé avant PIX de l'Ad5) du vecteur pAd.RSV.βGal, résultant en la substitution du gène βgal par le cDNA de l'Epo.

### 2.2. Construction de l'adénovirus recombinant défectif Ad.RSV.Epo

L'adénovirus Ad-RSV.Epo a été obtenu par recombinaison homologue in vivo entre l'adénovirus mutant Ad.d11324 [Thimmappaya et al., Cell 31 (1982) 543] et le vecteur pAd.RSV.Epo.

Pour cela, le plasmide pAd.RSV.Epo et l'adénovirus Ad.dll324 linéarisé par l'enzyme ClaI, ont été co-transfectés dans la lignée 293 en présence de phosphate de calcium, pour permettre la recombinaison homologue. Les adénovirus recombinants ainsi générés ont été sélectionnés par purification sur plaque. Après isolement, l'ADN de l'adénovirus recombinant a été amplifié dans la lignée cellulaire 293, conduisant à un surnageant de culture contenant l'adénovirus défectif recombinant non purifié ayant un titre d'environ 10¹⁰ pfu/ml.

Les particules virales sont généralement purifiées par centrifugation sur gradient de chlorure de césium selon les techniques connues (voir notamment Graham et al., Virology 52 (1973) 456). L'adénovirus Ad.RSV.Epo peut être conservé à -80°C dans 20 % de glycérol.

### 3. Préparation d'un implant comprenant des fibroblastes infectés par l'adénovirus recombinant Ad.RSV.Epo, du collagène et des fibres de polytétrafluoroéthylène (PTFE).

Les fibroblastes (10⁷ cellules/ml) préparés à partir de la peau de souris DBA2J selon le protocole décrit dans l'exemple 1.1. ont été infectés à confluence avec l'adénovirus Ad.RSV.Epo purifié sur gradient de chlorure de césium comme décrit dans l'exemple 2 (1 seul cycle d'infection). Deux conditions d'infection ont été utilisées, correspondant à deux titres : 10⁸ et 10⁹ pfu, soit une multiplicite d'infection de 10 et 100 respectivement.

24 heures après l'infection, les cellules ont été lavées, trypsinisées puis récoltées. Après un nouveau lavage, 4 implants ont été préparés selon le protocole décrit dans l'exemple 1.3., comprenant 10⁷ cellules chacun (2 implants pour chaque titre viral).

Les implants obtenus au bout de 72 heures de culture ont été prélevés puis implantés dans le péritoine de 2 souris DBA2J de 8 semaines selon le protocole décrit dans l'exemple 1.4. (2 implants par souris). La souris 1 contient les 2 implants réalisés avec les fibroblastes infectés avec un titre viral de 10⁹ pfu. La souris 2 contient les 2 implants réalisés avec les fibroblastes infectés avec un titre viral de 10⁸ pfu.

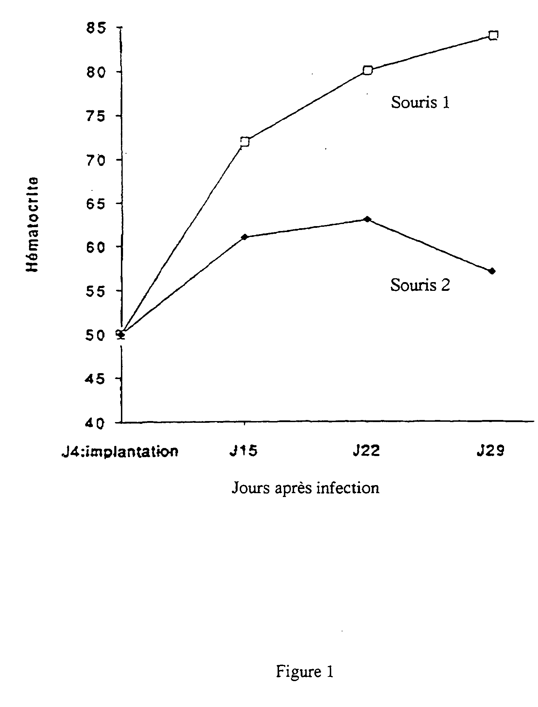

L'expression de l'Epo in vivo a été dosée dans le sang et son effet a été déterminé par mesure de l'élévation de l'hématocrite qui reflète l'action physiologique de l'Epo, c'est-à-dire l'augmentation du nombre de globules rouges. Pour cela, des échantillons de sang sont prélevés par ponctions au niveau du sinus rétroorbital au moyen d'un tube de microhématocrite, puis centrifugés dans une centrifugeuse de microhématocrite. Les résultats obtenus sont présentés sur la figure 1. Ils montrent clairement que les implants selon l'invention induisent une augmentation importante et dose-dépendante de l'hématocrite.

## Revendications

1. Composition destinée à être implantée dans un organisme **caractérisée en ce qu'**elle comprend des cellules modifiées par un adénovirus recombinant défectif comprenant une séquence d'ADN hétérologue codant pour un produit thérapeutique, un gélifiant, et un support sur lequel sont ancrées lesdites cellules.

2. Composition selon la revendication 1 **caractérisée en ce que** les cellules sont choisies parmi les fibroblastes, les cellules endothéliales, épithéliales, gliales, les hépatocytes, les kératinocytes et les myoblastes.

3. Composition selon la revendication 2 **caractérisée en ce que** les cellules sont des fibroblastes.

4. Composition selon l'une des revendications 1 à 3 **caractérisée en ce que** les cellules sont autologues vis-à-vis de l'organisme dans lequel elles sont destinées à être implantées.

5. Composition selon l'une des revendications 1 à 4 **caractérisée en ce que** l'adénovirus est un adénovirus d'origine humaine, canine ou mixte.

6. Composition selon la revendication 5 **caractérisée en ce que** l'adénovirus est obtenu à partir des adénovirus Ad2, Ad5 ou CAV2.

7. Composition selon l'une des revendications 1 à 6 **caractérisée en ce que** le produit thérapeutique est choisi parmi les ARN, peptides, polypeptides et protéines.

8. Composition selon la revendication 7 **caractérisée en ce que** la séquence d'ADN hétérologue comprend des signaux permettant la production et la sécrétion du produit thérapeutique.

9. Composition selon les revendications 7 et 8 **caractérisée en ce que** le produit thérapeutique est choisi parmi :
- les enzymes tels que la superoxide dismutase, la catalase, les amylases, les lipases, les amidases, la chymosine,
- les dérivés sanguins tels que la sérum-albumine, l'alpha- ou la béta-globine, le facteur VII, le facteur VIII, le facteur IX, le facteur de von Willebrand, la fibronectine, l'alpha-1 antitrypsine, l'insuline et ses variants,
- les lymphokines telles que les interleukines, les interférons, les facteurs de stimulation des colonies G-CSF, GM-CSF, M-CSF, SCF, le TNF, le TRF,
- les facteurs de croissance tels que l'hormone de croissance, l'érythropoiétine, l'hormone para-thyroïdienne, le FGF, l'EGF, le PDGF, le TGF, le BDNF, le NGF, le CNTF,
- les apolipoprotéines, ou encore des polypeptides antigéniques pour la réalisation de vaccins hépatite, cytomégalovirus, Epstein-Barr, herpes.

10. Composition selon la revendication 1 **caractérisée en ce que** le gélifiant est choisi parmi le collagène, la gélatine, les glycosaminoglycans, la fibronectine, et les lectines.

11. Composition selon la revendication 1 **caractérisée en ce que** le support est un support solide, non toxique et bio-compatible.

12. Composition selon la revendication 11 **caractérisée en ce que** le support est un support biologique.

13. Composition selon la revendication 12 **caractérisée en ce que** le support est choisi parmi le collagène réticulé, la poudre d'os, les polymères à base d'hydrates de carbone et les supports à base de calcaire.

14. Composition selon la revendication 13 **caractérisée en ce que** le support est choisi parmi les fibres de polytétrafluoroéthylène.

15. Procédé de préparation d'une composition selon l'une des revendications précédentes **caractérisé en ce que** l'on effectue les étapes suivantes :
a) on isole et cultive in vitro les cellules d'un échantillon de tissu,
b) on infecte les cellules obtenues en a) avec un adénovirus recombinant comprenant une séquence d'ADN hétérologue codant pour un produit thérapeutique,
c) on incube les cellules infectées avec un milieu contenant un gélifiant,
d) on dépose le mélange obtenu en c) sur un support, éventuellement après recouvrement de celui-ci par le gélifiant,
e) on incube le mélange obtenu en d) dans des conditions permettant la gélification du gélifiant et l'ancrage des cellules sur le support, et
f) on récupère la composition obtenue.

16. Utilisation d'une composition selon l'une des revendications 1 à 14, pour la préparation d'un médicament destiné être implanté au niveau de la cavité péritonéale, dans le tissu sous-cutané (région sus-pubienne, fosses iliaques ou inguinales, etc), dans un organe, un muscle, une tumeur, le système nerveux central, ou encore sous une muqueuse.

## Claims

1. Composition intended to be implanted in a body, **characterized in that** it comprises cells modified by a defective recombinant adenovirus comprising a heterologous DNA sequence coding for a therapeutic product, a gelling agent and a support to which the said cells are anchored.

2. Composition according to Claim 1, **characterized in that** the cells are chosen from fibroblasts, endothelial, epithelial and glial cells, hepatocytes, keratinocytes and myoblasts.

3. Composition according to Claim 2, **characterized in that** the cells are fibroblasts.

4. Composition according to one of Claims 1 to 3, **characterized in that** the cells are autologous with respect to the body in which it is intended to implant them.

5. Composition according to one of Claims 1 to 4, **characterized in that** the adenovirus is an adenovirus of human, canine or mixed origin.

6. Composition according to Claim 5, **characterized in that** the adenovirus is obtained from Ad2, Ad5 or CAV2 adenoviruses.

7. Composition according to one of Claims 1 to 6, **characterized in that** the therapeutic product is chosen from RNAs, peptides, polypeptides and proteins.

8. Composition according to Claim 7, **characterized in that** the heterologous DNA sequence comprises signals enabling the therapeutic product to be produced and secreted.

9. Composition according to Claims 7 and 8, **characterized in that** the therapeutic product is chosen from:
- enzymes such as superoxide dismutase, catalase, amylases, lipases, amidases or chymosin,
- blood derivatives such as serum albumin, alpha- or beta-globin, factor VII, factor VIII, factor IX, von Willebrand factor, fibronectin, alpha-1-antitrypsin or insulin and its variants,
- lymphokines such as interleukins, interferons, the colony stimulating factors G-CSF, GM-CSF, M-CSF, SCF, TNF or TRF,
- growth factors such as growth hormone, erythropoietin, parathyroid hormone, FGF, EGF, PDGF, TGF, BDNF, NGF or CNTF,
- apolipoproteins or alternatively antigenic polypeptides for the production of hepatitis, cytomegalovirus, Epstein-Barr or herpes vaccines.

10. Composition according to Claim 1, **characterized in that** the gelling agent is chosen from collagen, gelatin, glycosaminoglycans, fibronectin and lectins.

11. Composition according to Claim 1, **characterized in that** the support is a solid, non-toxic and biocompatible support.

12. Composition according to Claim 11, **characterized in that** the support is a biological support.

13. Composition according to Claim 12, **characterized in that** the support is chosen from crosslinked collagen, bone powder, carbohydrate-based polymers and limestone-based supports.

14. Composition according to Claim 13, **characterized in that** the support is chosen from polytetrafluoroethylene fibres.

15. Process for preparing a composition according to one of the preceding claims, **characterized in that** the following steps are performed:
a) cells from a tissue sample are isolated and cultured in vitro,
b) the cells obtained in a) are infected with a recombinant adenovirus comprising a heterologous DNA sequence coding for a therapeutic product,
c) the infected cells are incubated with a medium containing a gelling agent,
d) the mixture obtained in c) is deposited on a support, where appropriate after coating of the latter with the gelling agent,
e) the mixture obtained in d) is incubated under conditions permitting gelation of the gelling agent and anchorage of the cells to the support, and
f) the composition obtained is recovered.

16. Use of a composition according to one of Claims 1 to 14, for the preparation of a medicament intended to be implanted in the peritoneal cavity, in the subcutaneous tissue (suprapubic region, iliac or inguinal fossae, and the like), in an organ, a muscle, a tumour or the central nervous system, or alternatively under a mucosa.

## Patentansprüche

1. Zusammensetzung, die zum Implantieren in einen Organismus bestimmt ist, **dadurch gekennzeichnet, dass** sie Zellen umfasst, die von einem rekombinanten defektiven Adenovirus modifiziert wurden, umfassend eine heterologe DNA-Sequenz, die für ein therapeutisches Produkt kodiert, ein Gelatinierungsmittel, und einen Träger, auf dem die Zellen verankert sind.

2. Zusammensetzung nach Anspruch 1, **dadurch gekennzeichnet, dass** die Zellen ausgewählt sind aus Fibroblasten, Endothelzellen, Epithelzellen, Gliazellen, Hepatozyten, Keratinozyten und Myoblasten.

3. Zusammensetzung nach Anspruch 2, **dadurch gekennzeichnet, dass** die Zellen Fibroblasten sind.

4. Zusammensetzung nach einem der Ansprüche 1 bis 3, **dadurch gekennzeichnet, dass** die Zellen gegenüber dem Organismus autolog sind, in den sie implantiert werden sollen.

5. Zusammensetzung nach einem der Ansprüche 1 bis 4, **dadurch gekennzeichnet, dass** der Adenovirus ein Adenovirus humanen oder caninen oder gemischten Ursprungs ist.

6. Zusammensetzung nach Anspruch 5, **dadurch gekennzeichnet, dass** der Adenovirus aus den Adenoviren A^{d2}, Ad5 oder CAV2 erhalten wird.

7. Zusammensetzung nach einem der Ansprüche 1 bis 6, **dadurch gekennzeichnet, dass** das therapeutische Produkt ausgewählt ist aus RNA, Peptiden, Polypeptiden und Proteinen.

8. Zusammensetzung nach Anspruch 7, **dadurch gekennzeichnet, dass** die heterologe DNA-Sequenz Signale umfasst, die die Produktion und Sekretion des therapeutischen Produkts ermöglichen.

9. Zusammensetzung nach den Ansprüchen 7 und 8, **dadurch gekennzeichnet, dass** das therapeutische Produkt ausgewählt ist aus:
- Enzymen, wie etwa Superoxiddismutase, Catalase, Amylasen, Lipasen, Amidasen, Chymosin,
- Blutderivaten, wie etwa Serumalbumin, Alpha- oder Betaglobin, Faktor VII, Faktor VIII, Faktor IX, von-Willebrand-Faktor, Fibronektin, Alpha-1-Antitrypsin, Insulin und Varianten davon,
- Lymphokinen, wie etwa Interleukinen, Interferonen, Colony-stimulating-Faktoren G-CSF, GM-CSF, M-CSF, SCF, TNF, TRF,
- Wachstumsfaktoren, wie etwa Wachstumshormon, Erythropoietin, Nebenschilddrüsenhormon, FGF, EGF, PDGF, TGF, BDNF, NGF, CNTF,
- Apolipoproteinen, oder auch antigenen Polypeptiden zur Herstellung von Hepatitis-, Cytomegalovirus-, Epstein-Barr-, Herpes-Vakzinen.

10. Zusammensetzung nach Anspruch 1, **dadurch gekennzeichnet, dass** das Gelatinierungsmittel ausgewählt ist aus Collagen, Gelatin, Glykosaminoglykanen, Fibronektin, und Lektinen.

11. Zusammensetzung nach Anspruch 1, **dadurch gekennzeichnet, dass** der Träger ein fester, nicht toxischer und biokompatibler Träger ist.

12. Zusammensetzung nach Anspruch 11, **dadurch gekennzeichnet, dass** der Träger ein biologischer Träger ist.

13. Zusammensetzung nach Anspruch 12, **dadurch gekennzeichnet, dass** der Träger ausgewählt ist aus vernetztem Collagen, Knochenpulver, Polymeren auf der Basis von Kohlenhydraten und Trägern auf der Basis von Kalk.

14. Zusammensetzung nach Anspruch 13, **dadurch gekennzeichnet, dass** der Träger ausgewählt ist aus Polytetrafluorethylenfasern.

15. Verfahren zur Herstellung einer Zusammensetzung nach einem der vorhergehenden Ansprüche, **dadurch gekennzeichnet, dass** die folgenden Schritte ausgeführt werden:
a) Isolieren und Kultivieren der Zellen einer Gewebeprobe in vitro,
b) Infizieren der in a) erhaltenen Zellen mit einem rekombinanten Adenovirus, umfassend eine heterologe DNA-Sequenz, die für ein therapeutisches Produkt kodiert,
c) Inkubieren der infizierten Zellen mit einem Medium, das ein Gelatinierungsmittel enthält,
d) Abscheiden des in c) erhaltenen Gemischs auf einem Träger, gegebenenfalls nach dem Überziehen desselben mit dem Gelatinierungsmittel,
e) Inkubieren des in d) erhaltenen Gemischs unter Bedingungen, die das Gelieren des Gelatinierungsmittels und das Verankern der Zellen auf dem Träger ermöglichen, und
f) Rückgewinnen der erhaltenen Zusammensetzung.

16. Verwendung einer Zusammensetzung nach einem der Ansprüche 1 bis 14 zur Herstellung eines Medikaments, das zum Implantieren in die Peritonealhöhle, in das Unterhautzellgewebe (suprapubische Region, Fossa iliaca oder Fossa inguinalis usw.), in ein Organ, einen Muskel, einen Tumor, das zentrale Nervensystem oder auch unter eine Schleimhaut bestimmt ist.
